(19) Europäisches Patentamt
European Patent Office
Office européen des brevets

(11) Publication number: **0 473 145 A1**

# (12) EUROPEAN PATENT APPLICATION

(21) Application number: **91114479.8**

(51) Int. Cl.[5]: **A61K 37/02**, A61K 31/70

(22) Date of filing: **28.08.91**

(30) Priority: **28.08.90 US 574185**

(43) Date of publication of application:
**04.03.92 Bulletin 92/10**

(84) Designated Contracting States:
**AT BE CH DE DK ES FR GB GR IT LI LU NL SE**

(71) Applicant: **Bristol-Myers Squibb Company**
**345 Park Avenue**
**New York, N.Y. 10154(US)**

(72) Inventor: **McGregor, Donald N.**
**11 Oak Hill Drive**
**Clinton, Connecticut 06413(US)**
Inventor: **Davidson, Thomas J.**
**4929 Trawler Course**
**Liverpool, New York 13090(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

## (54) Compositions for inhibiting aminoglycoside nephrotoxicity.

(57) The use of specific polyaspartic acid-containing polymers as inhibitors of aminoglycoside nephrotoxicity.

EP 0 473 145 A1

The present invention is directed to the use of a selected subset of polyaspartic acid polymer protectants to inhibit the nephrotoxicity associated with the use of antibiotic aminoglycosides. The invention also covers pharmaceutical compositions comprising such protectants and aminoglycosides for clinical use when coadministration is desired.

Aminoglycoside antibiotics have been in use following the isolation of streptomycin, the first clinically useful aminoglycoside, in 1944. Although the aminoglycosides are particularly useful due to their rapid bactericidal action in infections by susceptible organisms, their use is limited to more severe, complicated infections primarily because of ototoxic and nephrotoxic side-effects. For this reason the aminoglycosides are considered to have a low therapeutic/risk ratio compared to other antibiotics used systemically.

Nephrotoxic effects from aminoglycoside usage are well documented in the literature. A recent description of aminoglycoside adverse reactions and precautions may be found in the 6th Edition of "Drug Evaluations", AMA and W.B. Saunders Co., Philadelphia, PA, pp. 1433-1435 (1986).

Several substances have been reported to be useful in inhibiting aminoglycoside-induced renal damage, particularly amino acid derivatives.

Meister, et al., in U.S. 4,758,551 reported $\gamma$-glutamyl amino acids to be nontoxic agents for use in combatting renal toxicity caused by nephrotoxic drugs. Shiokori, et al., in U.S. 4,757,066 disclosed the use of N-acylated amino acids in association with penem or carbapenem antibiotics to reduce renal toxicity caused by use of the antibiotic by itself. In WO 8804-925A (Tulane E. Fund Administration), biosynthetic precursors of oxidizable small peptides such as Glu-Cys or $(Gly\text{-}Cys\text{-}S)_2$ are among various agents reported to prevent the nephrotoxic effect of aminoglycosides.

Other agents, less related to the present invention, include inter alia D-glucarates (e.g. U.S. 3,928,583, 4,122,171) and amiloride (U.S. 4,654,325).

The most pertinent art would seem to be the disclosure by Williams, et al., in U.S. 4,526,888 describing the use of certain neutral and anionic polyamino acids to reduce aminoglycoside-induced nephrotoxicity. The specific polyamino acids taught by Williams, et al., were asparagine and aspartic acid polymers, and copolymers thereof.

The work of Williams, et al., reported in The J. of Pharmacology and Experimental Therapeutics, 237/3, 919-925 (1986) has been confirmed in rat model studies in other published reports. See: Gilbert, et al., The J. of Infectious Diseases, 159/5, 945-953 (May, 1989); Ramsommy et al., The J. of Pharmacology and Experimental Therapeutics, 250/1, 149-153 (1989).

These studies reporting the use of polyaspartic acid in animal models to treat aminoglycoside nephrotoxicity employed commercially available poly-L-aspartic acid (from Sigma Chemical Company). Commercially available poly-L-aspartic acid is characterized only by average molecular weight, which is determined by viscosity measurements or, more recently, by both viscosity and low angle laser light scattering techniques. From the name ascribed to the material (poly-L-aspartic acid) and from the method of synthesis given in the catalog ("...base-initiated polymerization of the corresponding N-carboxyanhydride."), these polymers can be expected to consist of repeating units of L-aspartic acid joined to each other via amide linkages involving the alpha carboxylic acid group of the aspartic acid with an average molecular weight as given for each sample. In addition, the experimental studies demonstrating protection were conducted using from at least 1.65 to 33 parts by weight of the polyaspartate per part of aminoglycoside.

When poly-L-aspartic acid lots from other sources, including custom synthesis, were examined by the inventors in a rat aminoglycoside nephrotoxicity model, unexplained variability in inhibitory potency was observed. In the course of determining the origin of such variability in inhibitory potency for poly-L-aspartic acid from other sources, the discovery was made that a subset of aspartic acid polymers, previously uncharacterized, was contained in the commercial poly-L-aspartic acid and that these contaminating polyaspartate substances possessed much greater inhibitory potency against the nephrotoxicity than did pure poly-L-aspartic acid (i.e., polyaspartic acid consisting predominantly of L-aspartic acid residues connected by amide links involving the alpha carboxylic acid group of the aspartic acid).

The present invention then is a result of the surprising discovery of polyaspartate polymeric materials with much higher inhibitory activity than the "pure" poly-L-aspartates in which this newly discovered component was incorporated. In the course of the present invention the improved subset of polyaspartate inhibitors has been characterized.

None of the above-listed substances or references reveal or suggest the previously uncharacterized polyaspartate materials which comprise the improved inhibitors of aminoglycoside nephrotoxicity as disclosed and claimed herein.

The invention results from the discovery that it was the presence of contaminating polyaspartate materials in commercial poly-L-aspartic acid that were responsible for the potent nephrotoxicity inhibition ascribed to poly-L-aspartic acid. Objectives of this invention then are to provide an improved method for

use with aminoglycoside antibiotics incorporating the newly characterized more potent polyaspartate inhibitors for the prevention or reduction of aminoglycoside induced nephrotoxicity and to provide improved pharmaceutical antibiotic compositions with much reduced nephrotoxicity than for the antibiotic agents alone, but requiring less of the inhibitory polymer.

The improved polyaspartic acid-containing polymers of the present invention are characterized as being water soluble; neutralized to physiologically acceptable pH; having an average molecular weight of about 600 to 9000 on a free acid basis; and having a "β-D factor" of at least 30. The "β-D factor" is derived by summation of the percentages of beta linkages and (D)-aspartic acid units in the polymers.

Research into the variability of different lots of poly-L-aspartic acid to inhibit aminoglycoside nephrotoxicity led to the discovery of a structural subset of polyaspartic acid-containing polymers with potent inhibitory activity compared with pure poly-L-aspartate. Polyaspartates can differ in structure not only due to incorporation of (D)- or (L)-monomeric units but also according to the specific amide linkage of units.

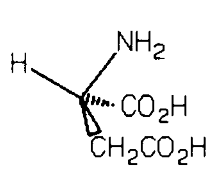

D- Isomer

(R)-(-)-aspartic acid

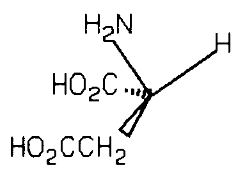

L- Isomer

(S)-(+)-aspartic acid

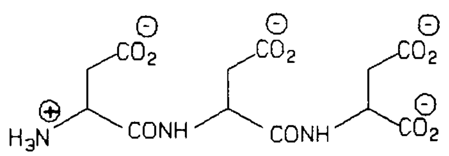

all α-linkage

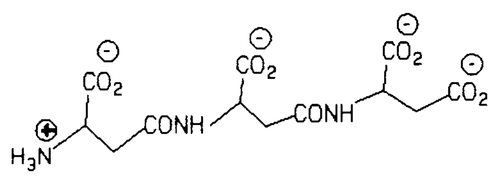

all β-linkage

The characterization of the potent polyaspartates of the present invention led to recognition of the role of D-residues and β-linkages in the preferred polyaspartates.

The improved method for inhibiting nephrotoxicity of an aminoglycoside antibiotic comprises the administration of at least one part by weight of a water soluble, partially neutralized polyaspartic acid polymer to two parts by weight of an aminoglycoside. The inhibitory polymer can be a homopolymer of aspartic acid or a copolymer comprised of aspartic acid and a lesser amount of a pharmaceutically acceptable comonomer. "Polymer" in the context of the present invention should be understood to apply to both homopolymers and copolymers of aspartic acid.

The polyaspartate polymers selected for use in the improved method have average molecular weights on a free acid basis from about 600 to 9000 and preferably about 800 to 6000; and possess a "β-D factor" of at least 30. The "β-D factor" is derived by summing the percentages of beta linkages and (D)-aspartic acid units in the polymers. Any aspartic acid polymer can have from 0 to 100% of beta linkages and from 0 to 100% of (D)-residues. Thus the "β-D factor" can range from 0 to 200 for these polymers. While polyaspartates with a "β-D factor" less than 30 have relatively poor nephrotoxicity inhibition potency, those with various "β-D factor" values greater than 30 appear to be comparably effective as nephrotoxicity inhibitors. Thus, it is not necessary to select a polyaspartate with the maximum "β-D factor" of 200 (i.e., "pure" β-D-polyaspartate). This is a potential economic advantage since some of the most simple, efficient and inexpensive aspartic acid polymerization processes inherently produce a polymer which contains a mixture of D and L residues and α and β linkages.

The administration of the improved polyaspartate inhibitor can be done separately but concurrently with the administration of a therapeutically effective amount of an aminoglycoside antibiotic. The administration of inhibitors may also be before or after and may also be less frequent. The inhibitor and antibiotic can also be combined and coadministered in a pharmaceutically acceptable carrier.

The improved polyaspartates have been neutralized to provide a physiologically acceptable pH when dissolved in the administration vehicle. This pH is in the general range of about 6 to 8, preferably about 7. The neutralization is accomplished with a suitable base such as NaOH, KOH, $NH_4OH$, etc., thereby increasing water solubility of these polyanionic aspartic acid polymers. When NaOH is used as the neutralization agent, approximately 70-80% of the carboxylate moieties are in form of the sodium salt. Polyaspartic acid as the free acid lacks stability.

The molecular weights of the polyaspartate inhibitors vary with the sodium salt content.

| Aspartic Acid Units | Molecular Weight | | |
|---|---|---|---|
| | Free Acid | 70% Na Salt | 100% Na Salt |
| 10 | 1169 | 1338 | 1411 |
| 12 | 1399 | 1599 | 1685 |
| 20 | 2320 | 2643 | 2781 |
| 40 | 4622 | 5253 | 5523 |
| 80 | 9225 | 10472 | 11006 |

The aminoglycoside antibiotic which can be employed in conjunction with the nephrotoxicity inhibiting polymers of the invention is any aminoglycoside antibiotic. Examples of such aminoglycoside antibiotics include kanamycin (Merck Index 11th ed. #5161), gentamicin (Merck Index 11th ed. #4283), amikacin (Merck Index 11th ed. #416), dibekacin (Merck Index 11th ed. #2987), tobramycin (Merck Index 11th ed. #9413), streptomycin (Merck Index 11th ed. #8786/8787), paromomycin (Merck Index 11th ed. #6989), sisomicin (Merck Index 11th ed. #8498), isepamicin (Merck Index 11th ed. #4989), neomycin (Merck Index 11th ed. #6369), fortimicin (Merck Index 11th ed. #4165 and netilmicin (Merck Index 11th ed. #6389), all known in the art. The useful antibiotics include the several structural variants of the above compounds (e.g. kanamycin A, B and C; gentamicin A, $C_1$, $C_{1a}$, $C_2$ and D; neomycin B and C and the like).

All aminoglycoside antibiotics tested to date accumulate in renal tissue and possess a certain nephrotoxic potential [Luft et al., J. Inf. Dis., 138(4): 541-595 (1978)]. Thus, the present invention is useful with any aminoglycoside antibiotic. In usual practice, pharmaceutically acceptable acid addition salts of these aminoglycoside antibiotics are employed.

For the purpose of this disclosure, the terms "pharmaceutically acceptable acid addition salt" shall mean a mono or poly salt formed by the interaction of one molecule of the aminoglycoside antibiotic with one or more moles of a pharmaceutically acceptable acid. Included among those acids are acetic, hydrochloric, sulfuric, maleic, phosphoric, nitric, hydrobromic, ascorbic, malic and citric acid, and those other acids commonly used to make salts of amine-containing pharmaceuticals.

In a special application of this invention, the carboxylic acid groups of the polyaspartic acid can be utilized to form acid addition salts of the amine groups of the aminoglycoside. If necessary, the resulting aminoglycoside-polyaspartic acid salt can be neutralized with either a pharmaceutically acceptable base or a pharmaceutically acceptable acid.

The renal toxicity inhibitors of this invention conjointly used with the nephrotoxic aminoglycosides are water-soluble salt forms of polymers of polyaspartic acid, including homopolymers and pharmaceutically acceptable copolymers of polyaspartic acid with lesser amounts of comonomers. The useful polyaspartate polymers possess physical characteristics that provide the following prerequisite polymer properties:

- water soluble: all or a sufficient number of the organic acid moieties (i.e. carboxylic acid groups) are in ionic form due to salt formation with a water-soluble, pharmaceutically acceptable cation ($Na^+$; $K^+$; $NH_4^+$; and the like) so that the polymer is readily water-soluble. Not every organic acid moiety in the polymer need be in ionic salt form.
- polyanionic (by virtue of ionized organic acid functional moieties, e.g. carboxylate).
- sufficiently small molecular weight to pass through the glomerulus;
- large enough to efficiently complex the aminoglycoside under the appropriate physiological conditions;
- sufficiently stable (resistant to host metabolism).

The measured molecular weights are "average" molecular weights of the polydisperse polyaspartates. The techniques and different methods of determining polymer molecular weights and structural characterization of the polymers are known to those skilled in the polymeric chemical arts.

The polyaspartate inhibitors of this invention and aminoglycosides can be conjointly used in a variety of modes. The aminoglycoside antibiotic and the inhibitory polymer can be combined into a single dosage

unit, preferably with a pharmaceutically acceptable carrier, for example, a cosolution or dispersion in an inert pharmaceutically acceptable solvent or dispersing agent or the like. Typically, pharmaceutically acceptable carriers can be any of those heretofore employed or compatible with the aminoglycoside antibiotic.

Alternatively, the polyaspartate inhibitor can be separately formulated with pharmaceutically acceptable materials and administered separately; either concurrently with the aminoglycoside antibiotic or before or after administration of the aminoglycoside antibiotic.

Another aspect of this invention then concerns pharmaceutical formulations comprising either just polyaspartic acid-containing polymers of the instant invention as the active ingredient or the polyaspartic acid-containing polymer in combination with an aminoglycoside as active ingredients, associated with one or more pharmaceutically acceptable carriers, excipients or diluents therefor.

The mode of administration, the dosage and frequency of dosage is governed by the mode of administration and dosage considerations conventionally employed with the aminoglycoside antibiotic. Thus, for example, various of the combinations of the invention can be administered intramuscularly or intravenously, or otherwise, as dictated by medical and pharmacological practice related to the desired use of the particular antibiotic employed.

The amount of polyaspartate inhibitor employed in conjunction with the aminoglycoside antibiotic is an aminoglycoside antibiotic nephrotoxicity reducing amount. The amount varies depending upon the aminoglycoside employed. Typically, the amount of improved polyaspartate inhibitor employed is at least about one part by weight per two parts of the aminoglycoside antibiotic. The upper limit is also influenced by practical weight and cost considerations. Amounts as high as 33 parts of the improved polyaspartate per part of antibiotic have been shown to be effective, but appear to be unnecessary as much lower levels appear to provide excellent inhibition. A preferred range of improved polyaspartate inhibitor to aminoglycoside ratio is from about 0.5 to 4 parts of inhibitor per part by weight of antibiotic.

Due to the greater inhibitory potency of the improved polyaspartate on a weight basis, less of the polymer is required to achieve the protective effect. This is advantageous not only from an economic view, but therapeutically as well since a decreased amount of any medication will reduce the probability that a undesired reaction to that medication will occur.

It may be seen then that by employing the improved water-soluble, partially neutralized, polyaspartate inhibitors of the specified average molecular weight and "$\beta$-D factor" value, in a pharmaceutically acceptable salt form, the two objectives of the invention can be met.

1. A method for preventing or reducing nephrotoxicity of an aminoglycoside antibiotic is disclosed herein and comprises the administration of an effective dose of a water-soluble salt form of a polyaspartate mixture with an average molecular weight from about 600 to about 9,000. The polyaspartates are comprised of polymerized units of aspartic acid: either singly (homopolymer) or mixed with lesser amounts of a pharmaceutically acceptable comonomer (copolymer).

2. A second objective provides improved aminoglycoside compositions having reduced nephrotoxicity as a result of incorporation of the improved nephrotoxicity-inhibiting polyaspartates of the present invention. These pharmaceutical compositions comprise antibiotic-effective amounts of aminoglycosides, effective nephrotoxicity-inhibiting amounts of the improved polyaspartate polymers as described herein, and pharmaceutically acceptable carriers and/or vehicles which would be familiar to one skilled in the pharmaceutical arts. The actual amounts of aminoglycosides employed will range from those given in standard references for prescription drugs (e.g. "Physicians Desk Reference", 42nd Edition (1988); "Drug Evaluations" AMA, 6th Edition (1986)). Because of the reduced risk of nephrotoxicity with these compositions, it may be possible to give larger doses of the aminoglycoside than is normally recommended.

The nephrotoxicity-inhibiting effects of the polyaspartate polymers of this invention were demonstrated in a rat model of gentamicin-induced nephrotoxicity. A ten day period of gentamicin dosing (50mg/kg/day) reproducibly results in severe nephrotoxicity as shown by increased serum urea nitrogen (BUN) and creatinine levels as well as by microscopic kidney evaluation. Coadministering an improved polyaspartate of this invention prevented or significantly ameliorated gentamicin-induced nephrotoxicity.

Example I

Study No. I:

Treatment groups consisting of five male Fischer 344 rats received two daily subcutaneous injections of:

a. 7.36% NaCl in water, 1.0 ml/kg
b. 25 mg/kg of gentamicin in 7.36% NaCl-water
c. 25 mg/kg of gentamicin and 25 mg/kg of sodium polyaspartate
d. 25 mg/kg of gentamicin and 200 mg/kg of sodium polyaspartate

Additional treatment groups were used for the c. and d. treatment protocols in order to study the "β-D factor". Five polyaspartate samples with differing "β-D factor" values were studied.

Properties of various samples of sodium polyaspartate in Study No. I:

| sample No. | % beta linkages | % D residues | β-D Factor (% beta + % D) |
|---|---|---|---|
| 1 | 5 | 9 | 14 |
| 2 | 8 | 10 | 18 |
| 3 | 54 | 20 | 74 |
| 4 | 63 | 22 | 85 |
| 5 | 62 | 24 | 86 |

Thus there was a total of 5 treatment groups per each dosing protocol c. and d.

The injections were continued for 10 days. On day 11, the rats were sacrificed and the kidneys were subjected to histopathologic examination. In the following Table, the number of rats in each of the following histopathologic category groups is recorded:

A. Marked and moderate degenerative change
B. Moderate necrotic change
C. Marked and moderate regenerative change
D. Moderate tubular degeneration and necrosis
E. Marked and moderate tubular regeneration and hypertrophy

Table I

| Treat Group | Histopath. Category A | Histopath. Category B | Histopath. Category C | Histopath. Category D | Histopath. Category E | Sum |
|---|---|---|---|---|---|---|
| a | 0 | 0 | 0 | 0 | 0 | 0 |
| b | 5 | 4 | 5 | 4 | 5 | 23 |
| c(1) | 5 | 1 | 5 | 3 | 5 | 19 |
| d(1) | 2 | 1 | 2 | 1 | 2 | 8 |
| c(2) | 5 | 0 | 5 | 3 | 5 | 18 |
| d(2) | 3 | 0 | 3 | 0 | 3 | 9 |
| c(3) | 3 | 0 | 3 | 0 | 4 | 10 |
| d(3) | 0 | 0 | 0 | 0 | 0 | 0 |
| c(4) | 2 | 1 | 2 | 1 | 3 | 9 |
| d(4) | 0 | 0 | 0 | 0 | 0 | 0 |
| c(5) | 4 | 0 | 5 | 0 | 5 | 14 |
| d(5) | 0 | 0 | 0 | 0 | 0 | 0 |

Conclusions:

Samples 1 and 2 gave only partial protection at the high-dose level (protocol d), while samples 3, 4, and 5 gave complete protection at the high-dose level. At the low-dose level (protocol c), samples 3, 4, and 5 gave partial protection, while samples 1 and 2 gave very little protection.

Example 2

Study No. II:

The treatment groups were the same as in Study No. I, except that four F344 rats were in each treatment group.
Properties of various samples of sodium polyaspartate in Study No. II:

| Sample No. | % beta linkages | % D residues | β-D Factor (% beta + % D) |
|---|---|---|---|
| 6 | 11 | 19 | 30 |
| 7 | 71 | 23 | 94 |
| 8 | 75 | 50 | 125 |
| 9 | 77 | 49 | 126 |

The kidneys of the test rats were subjected to histopathologic examination, and each kidney was assigned a score of 4 = severe, 3 = moderate, 2 = mild, 1 = minimal, or 0 = not present in each of the histopathologic categories of degeneration (DEG), necrosis (NEC), and regeneration (REG). In the following Table, the mean score for each group in each of the histopathologic categories is listed.

Table II

| Treat. Group | Mean DEG score | Mean NEC score | Mean REG score | Sum |
|---|---|---|---|---|
| a | 0 | 0 | 0.1 | 0.1 |
| b | 4.0 | 3.0 | 3.6 | 10.6 |
| c(6) | 1.5 | 0.5 | 1.0 | 3.0 |
| d(6) | 0 | 0 | 0 | 0 |
| c(7) | 1.9 | 1.2 | 2.0 | 5.1 |
| d(7) | 0 | 0 | 0 | 0 |
| c(8) | 2.0 | 1.2 | 1.8 | 5.0 |
| d(8) | 0 | 0 | 0 | 0 |
| c(9) | 1.5 | 0.2 | 0.6 | 2.3 |
| d(9) | 0 | 0 | 0 | 0 |

Conclusions:

All samples gave complete protection at the high dose level (protocol d) and partial protection at the low dose level (protocol c).

Synthesis of Polyaspartic Acid Polymers

The synthesis of polymers of aspartic acid can be carried out by a variety of methods familiar to those skilled in the art of polymer chemistry. The following is a summary of the most important of these methods which can be used for preparation of the polyaspartic acid polymer protectants of this invention.

1. Aspartic acid polymers of defined length, D- and L-isomer ratio, α- and β-linkages, and copolymer content can be prepared by normal peptide synthesis techniques in which monomer units are added sequentially to the growing peptide chain. The preferred methodology for this approach is solid phase peptide synthesis which has been widely described, for example, in J.M. Stewart and J.D. Young, Solid Phase Peptide Synthesis, 2nd Edition, Pierce Chemical Company, 1984.

2. An extensively investigated method for the preparation of homopolymers and copolymers of amino acids employs an N-carboxy-α-amino acid anhydride intermediate (see, for example, E. Katchalski, Adv. Prot. Chem., 1951, 6, 123). When this technique is applied to aspartic acid, the β-carboxylic acid group as blocked as an ester (often a benzyl ester), the N-carboxy anhydride intermediate is formed, a base-initiated polymerization is carried out, and the blocking groups are removed to form the polyaspartic acid. By modifying the starting material and the conditions of the polymerization and deblocking conditions, it is possible to obtain samples of polyaspartic acid of differing molecular weight, D-and L-isomer content, and α- and β-linkage content. V. Saudek et al., Biopolymers, 1981, 20, 1615, have shown that modification of the deblocking conditions can afford β-linkage contents ranging from <10% to 75%,

and D-isomer content ranging from 0% to 50% (the D-isomer content can be further varied, of course, by using starting materials containing the desired D-isomer percentage). In this study, the β-linkage content was determined by $^{13}C$-nmr (see H. Pivcova et al., Biopolymers, 1981, 20, 1605) (β-linkage content can also be determined by potentiometric tritation - see V. Saudek and J. Drobnik, Polymer Bull., 1981, 4, 473), the D-isomer content was determined by hydrolysis and optical rotation, and the molecular weights of the polymers were determined by viscosity measurements (a preferred method for the determination of polymer molecular weight is low angle laser light scattering, LALLS - see P.J. Wyatt et al., Amer. Laboratory, 1988, 20, 86). The polymer molecular weight can be controlled by adjustment of the amount of polymerization initiator and the polymerization temperature - see E. Katchalski, loc. cit. This method can also be used to prepare copolymers by carrying out the polymerization reaction with a mixture of N-carboxy anhydrides (see, for example, Y. Kobayashi et al., Macromolecules, 1977, 10, 1271).

3. A method which is especially effective for the preparation of aspartic acid polymers is thermal condensation (see J. Kovacs et al., Experientia, 1953, 9, 459; J. Kovacs et al., J. Org. Chem., 1961, 26, 1084). In this technique, aspartic acid or a derivative thereof is heated to form polysuccinimide, which is then hydrolyzed to form polyaspartic acid. The conditions of these transformations lead to polyaspartic acids containing D-isomer ranging from approximately 20% to approximately 80% (depending on whether the D- or the L-isomer is used as starting material - see E. Kokufuta et al., Bull. Soc. Chem. Japan, 1978, 51, 1555) and β-linkage content ranging from approximately 50% to approximately 80% (V. Saudek and J. Drobnik, Polymer Bull., 1981, 4, 473; H. Picova et al., Polymer, 1982, 23, 1237). The molecular weight of the polymer can be controlled by modifications in the polymerization conditions - see E. Kokufuta et al., loc. cit.; P. Neri et al., J. Med. Chem., 1973, 16, 893. This method can also be used for the preparation of copolymers of aspartic acid - see S.W. Fox and K. Harada, Analytical Methods in Protein Chemistry, p. Alexander and H.P. Lundgren, eds., Pergamon Press, 1961, 4, 129.

4. Other methods have been described for the preparation of polyaspartic acids. T. Munegumi et al., Bull. Soc. Chem. Japan, 1989, 62, 2748, have described the polymerization of aspartic acid anhydride formed from N-carbobenzyloxyaspartic acid anhydride. The preparation of poly-β-L-aspartic acid by the polymerization of α-benzyl-β-N-succinimidyl-L-aspartate has been described (P.M. Hardy et al., J. Chem. Soc. Perkin I, 1972, 605).

The following examples are illustrative of the preferred methods of preparation of the improved polyaspartate inhibitors of the present invention. These examples are intended only for the purpose of illustration and are not to be construed as limiting the invention in sphere or scope.

Example 3

A. N-carboxy anhydride derivative of β-benzyl-L-aspartate.

To a suspension of β-benzyl-L-aspartate (2.23 g, 10 mmol) in dry THF (30 mL) was added trichloromethyl chloroformate (0.67 mL, 5.5 mmol) at 45 °C under an argon atmosphere. The clear solution was stirred for 1 h at 45 °C, solvent distilled off under reduced pressure at 30 °C, ethyl acetate (20 mL) was added to the residue and filtered. Hexane (50 mL) was added to the filtrate and the bulky precipitate was separated, washed with hexane (2 x 50 mL) and dried in vacuum to a white fluffy solid (2.3 g, 93%). IR (nujol) 1730, 1800 $cm^{-1}$. $^1H$ NMR ($CDCl_3$) δ 7.37-7.33 (m, 5H, phenyl), 6.18 (br s, 1H, NH), 5.17 (s, 2H, $OCH_2$ ester), 4.58 (ddd, 1H, H-1, $J_{1-6}=3.27$ Hz, $J_{1-6'}=9.33$ Hz, J = 1.17 Hz, (not shown after $D_2O$ treatment), 3.08 (dd, 1H, H-6, $J_{6,6'}=17.7$ Hz), 2.82 (dd, 1H, H-6'). $^1H$ NMR (acetone-$d_6$) δ 7.41-7.29 (m, 5H, phenyl), 5.16 (s, 2H, $OCH_2$ ester),4.81 (t, 1H, H-1, $J_{1-6}=J_{1-6'}=4.77$ Hz), 3.14 (dd, 1H, H-6, $J_{6-6'}=17.76$ Hz). $^{13}C$ NMR ($CDCl_3$) δ 169.36, 168.24 (2 x C=O), 151.29 (C-3), 134.67 (ipso-phenyl), 128.85, 128.79, 128.56 (phenyl), 67.81 ($OCH_2$, ester), 53.86 (C-1), 36.09 (C-6). Mass spectrum (FAB, glycerol) m/e (relative intensity) 342 (M+H+glycerol, 33), 298 (342-$CO_2$, 40), 270 (298-CO, 78), 250 (M+H, 21), 185 (2 glycerol + H, 100).

B. Dibenzyl L-aspartate.

A mixture of L-aspartic acid (33.27 g, 0.25 mol), benzyl alcohol (100 mL) and p-toluenesulfonic acid monohydrate (48.5 g, 0.255 mol) in benzene (50 mL) was refluxed for 18 h with a Dean Stark trap to azeotrope $H_2O$. Benzene (200 mL) and anhydrous ether (600 mL) were added to the mixture and left at 4 °C. The crystalline p-toluenesulfonic acid salt was filtered, washed with anhydrous ether and recrystallized from a mixture of methanol and ether. The salt (15 g, 30.8 mmol) was dissolved in a 1:1 mixture of acetone and $H_2O$ (300 mL) and made basic with portionwise addition of solid $K_2CO_3$. The mixture was extracted with

EtOAc (2 x 150 mL), the combined organic phases were washed with $H_2O$ (100 mL), dried over anhydrous $Na_2SO_4$ and concentrated to afford an oil (9.5 g, 98%). $^1H$ NMR ($CDCl_3$) $\delta$ 7.37-7.24 (m, 10H, phenyl), 5.11, 5.08 (s, 4H, $OCH_2$ ester), 3.84 (dd, 1H, $C_\alpha H$ $J_{\alpha,\beta} = 5.0$ Hz, $J_{\alpha,\beta'} = 6.9$ Hz), 2.86 (dd, 1H, $C_\beta H$, $J_{gem} = 16.5$ Hz), 2.75 (dd, 1H, $C_\beta H'$). $^{13}C$NMR ($CDCl_3$) $\delta$ 173.79, 170.72 (2 x C=O), 135.4, 135.3 (2 x ipso-phenyl), 128.39, 128.21, 128.09 (phenyl), 66.84, 66.39 (2 x $OCH_2$), 51.11 ($C_\alpha$), 38.76 ($C_\beta$).

C. General procedure for the polymerization of NCA.

To a solution of the N-carboxy anhydride derivative of $\beta$-benzyl-L-aspartate (10 g, 40 mmol) in dry $CH_3CN$ (200 mL) was added a solution of dibenzyl-L-aspartate (0.25 g, 0.8 mmol) in 5 mL of the same solvent under an argon atmosphere. The mixture was vigorously stirred at room temperature for 120 h under argon to flush off the $CO_2$ formed during the reaction. The precipitate was separated, washed successively with $CH_3CN$ (200 mL) and n-hexane (200 mL), and vacuum dried over $P_2O_5$ (4.83 g, 59%). $^1H$ NMR (DMSO: $CD_2Cl_2$) $\delta$8.5-7.9 (br, 1H, NH), 7.4-7.1 (m, 5H, phenyl), 5.0 (s, 2H, $OCH_2$ ester), 4.6-4.4 (br, 1H, $C_\alpha H$), 3.0-2.6 (m, 2H, $C_\beta H,H'$). $^{13}C$ NMR ($CD_2Cl_2$) $\delta$ 172.18, 171.12 (C=O), 136.45, 136.18 (ipso-phenyl), 128.84, 128.47 (phenyl), 66.74 ($OCH_2$, ester), 51.76 ($C_\alpha$), 34.17 ($C_\beta$).

D. The polymerization product from Section C (0.3 g), ethyl methyl sulfide (6.5 mL) and p-cresol (1 g) were placed in the reaction vessel and connected to the HF line. The vessel was cooled to -78 °C for 0.5 h and the line was evacuated briefly. HF was distilled into the vessel to a pre-marked level (2.5 mL). The reaction was then quickly equilibrated to 0 °C by ice bath and allowed to stir vigorously for 2 h. HF and ethyl methyl sulfide were distilled off carefully at 0 °C under reduced pressure. The residue was dissolved in 10% aqueous $(NH_4)_2CO_3$ (20 mL), extracted with EtOAc (3 x 50 mL) and freeze dried to a gum (0.35 g). The gum was dissolved in $H_2O$ and passed through a column of mixed bed ion exchange resins (Dowex 50W-X8, ionic form $H^+$ and Rexyn 203 (OH), ionic form $OH^-$) to remove the sulfonium salt by-product. Mixing appropriate fractions and freeze drying gave the pure deblocked acid (0.06 g, 43%) which was converted to the sodium salt by passing through a column of ion exchange resin, Dowex 50W-X8 (ionic form $Na^+$). $^1H$ NMR ($D_2O$ $\delta$ 4.66 (t, 1H, $C_\alpha H$, $J_{\alpha,\beta H} = 6.44$ Hz), 2.85, 2.72 (m, 2H, $C_\beta H$, H', $J_{gem} = 16.8$ Hz): $^{13}C$ NMR ($D_2O$) $\delta$ 176.46, 172.82 (C=O), 51.09 ($C_\alpha$), 37.31 ($C_\beta$). This sodium polyaspartate product corresponds to Example No. 1 and has a $\beta$-D Factor of 14.

Example 4

Sodium polyaspartate (thermal polymerization process).

A suspension of finely powdered D,L-aspartic acid (30 g, 0.225 mol) in tetralin (300 mL) was refluxed for 96 h to remove the $H_2O$ by azeotropic distillation. The tawny precipitate obtained was separated, washed with ether and triturated with saturated $NaHCO_3$ solution (3 x 100 mL). The initial granular product turned into a fine powder with each trituration. The product was either filtered and/or centrifuged depending on the degree of difficulty encountered during each filtration. The product was washed with $H_2O$, 1% HCl and finally with $H_2O$ until the filtrate was salt free ($AgNO_3$ test). The yield of the light yellow anhydropolyaspartic acid was 16 g. A suspension of this material (9.5 g) in $H_2O$ (30 mL) was treated with a 2M NaOH solution (~45 mL) dropwise while stirring the mixture to attain a pH of ~10. The mixture was heated to ~95 °C for 15 min. to ensure complete hydrolysis and the pH was finally readjusted to ~8 with 1N HCl. The solution was filtered and passed through a column of Sephadex G-25 (dia. 5 cm, height 100 cm) in order to achieve partial fractionation and complete desalting. After analyzing the fractions by HPLC, they were mixed to give two large fractions which were subsequently freeze dried. $^1H$ NMR ($D_{20}$) $\delta$ 4.68 (br s, $C_\alpha H$, $\alpha$-peptide, 30%), 4.49 (br s, $C_\alpha H$, $\beta$-peptide, ~70%), 2.77 (br m, 2H, $C_\beta H$, H'). $^{13}C$ NMR ($D_2O$, pD ~10) $\delta$ 177.81 (C=O, $\beta$-peptide), 177.37 (C=O, $\alpha$-peptide), 172.72, 172.62 (CONH, $\alpha$-peptide), 172.02, 171.89 (CONH, $\beta$-peptide), 51.83 ($C_\alpha\beta$-peptide), 51.38 ($C_\alpha$, $\alpha$-peptide), 39.0 ($C_{\beta,}\alpha$-peptide), 37.69 ($C_\beta$, $\beta$-peptide). This sodium polyaspartate product corresponds to Sample No. 8 and has a $\beta$-D Factor of 125.

**Claims**

**1.** A polyaspartic acid-containing polymer, in pharmaceutically acceptable salt form which is characterized as possessing:

a) water solubility;

b) a pharmaceutically acceptable pH when dissolved in the administration vehicle;

c) an average molecular weight of about 600 to 9000 on a free acid basis; and

d) a "$\beta$-D factor" of at least 30, the "$\beta$-D factor" being derived by summation of the percentages of beta linkages and (D)-aspartic acid units in the polymers.

**2.** A polymer of Claim 1 which is a polyaspartic acid homopolymer of aspartic acid.

**3.** A polymer of Claim 1 which is a copolymer of aspartic acid and a lesser amount of a pharmaceutically acceptable comonomer.

**4.** A pharmaceutical formulation which comprises as an active ingredient a polyaspartic acid-containing polymer, as claimed in any one of Claims 1 to 3, associated with one or more pharmaceutically acceptable carriers, excipients or diluents therefor.

**5.** A pharmaceutical formulation which comprises, as active ingredients, two parts by weight of an aminoglycoside antibiotic and at least one part by weight of a polyaspartic acid-containing polymer, as claimed in any one of Claims 1 to 3, associated with one or more pharmaceutically acceptable carriers, excipients or diluents therefor.

**6.** A formulation of Claim 5 wherein the polyaspartic acid-containing polmer is a homopolymer of aspartic acid

**7.** A formulation of Claim 5 wherein the aminoglycoside is gentamycin or amikacin.

**8.** A formulation of any of Claims 4 to 7 in unit dosage form.

**9.** A kit adapted for simultaneous or sequential administration which comprises, in a pharmaceutically acceptable form, (1) an aminoglycoside antibiotic and, (2) a polyaspartic acid-containing polymer as defined in any one of Claims 1 to 3.

**10.** A polyaspartic acid-containing polymer as claimed in any of Claims 1 to 3 for use in inhibiting nephrotoxicity of an aminoglycoside antibiotic.

**Claims for the following Contracting State:ES**

**1.** A process for preparing a polyaspartic acid-containing polymer characterized as possessing:
a) water solubility;
b) a pharmaceutically acceptable pH when dissolved in the administration vehicle;
c) an average molecular weight of about 600 to 9000 on a free acid basis; and
d) a "$\beta$-D factor" of at least 30, the "$\beta$-D factor" being derived by summation of the percentages of beta linkages and (D)-aspartic acid units in the polymers,
the synthetic process comprising amide bond formation between aspartic acid units themselves to form homopolymers or between aspartic acid units and units of a pharmaceutically acceptable comonomer to form copolymers; this followed by neutralization of the acidic polymer with a pharmaceutically acceptable base to bring the pH within the range of about pH6 to pH8.

**2.** A process as claimed in Claim 1 for preparing a polyaspartic acid homopolymer.

**3.** A process as claimed in Claim 1 for preparing a copolymer of aspartic acid and a lesser amount of a pharmaceutically acceptable comonomer.

**4.** A process for preparing a kit adapted for simultaneous or sequential administration which comprises combining in a pharmaceutically acceptable form, (1) an aminoglycoside antibiotic and, (2) a polyaspartic acid-containing polymer as defined in any one of Claims 1 to 3.

**5.** A process for preparing a pharmaceutical formulation which comprises incorporating a polyaspartic acid-containing polymer as defined in any one of Claims 1 to 3 into one or more pharmaceutically acceptable carriers, excipients or diluents.

**6.** A process for preparing a formulation by admixture of two parts by weight of an aminoglycoside

antibiotic with at least one part by weight of a polyaspartic acid-containing polymer, as defined in Claim 1, in association with one or more pharmaceutically acceptable carriers, excipients or diluents therefor.

**7.** A process as claims in Claim 6 wherein the aminoglycoside is gentamicin or amicacin.

**8.** A process as claimed in anyone of Claims 5 to 7 for preparing the formulation in unit dosage form.

**Claims for the following Contracting State:GR**

**1.** A process for preparing a polyaspartic acid-containing polymer characterized as possessing:
a) water solubility;
b) a pharmaceutically acceptable pH when dissolved in the administration vehicle;
c) an average molecular weight of about 600 to 9000 on a free acid basis; and
d) a "$\beta$-D factor" of at least 30, the "$\beta$-D factor" being derived by summation of the percentages of beta linkages and (D)-aspartic acid units in the polymers.
the synthetic process comprising amide bond formation between aspartic acid units themselves to form homopolymers or between aspartic acid units and units of a pharmaceutically acceptable comonomer to form copolymers; this followed by neutralization of the acidic polymer with a pharmaceutically acceptable base to bring the pH within the range of about pH6 to pH8.

**2.** A process as claimed in Claim 1 for preparing a polyaspartic acid homopolymer.

**3.** A process as claimed in Claim 1 for preparing a copolymer of aspartic acid and a lesser amount of a pharmaceutically acceptable comonomer.

**4.** A process for preparing a kit adapted for simultaneous or sequential administration which comprises combining in a pharmaceutically acceptable form, (1) an aminoglycoside antibiotic and, (2) a polyaspartic acid-containing polymer as defined in any one of Claims 1 to 3.

**5.** A process for preparing a pharmaceutical formulation which comprises incorporating a polyaspartic acid-containing polymer as defined in any one of Claims 1 to 3 into one or more pharmaceutically acceptable carriers, excipients or diluents.

**6.** A process for preparing a formulation by admixture of two parts by weight of an aminoglycoside antibiotic with at least one part by weight of a polyaspartic acid-containing polymer, as defined in Claim 1, in association with one or more pharmaceutically acceptable carriers, excipients or diluents therefor.

**7.** A process as claims in Claim 6 wherein the aminoglycoside is gentamicin or amicacin.

**8.** A process as claimed in anyone of Claims 5 to 7 for preparing the formulation in unit dosage form.

**9.** A polyaspartic acid-containing polymer as claimed in any of Claims 1 to 8 for use in inhibiting nephrotoxicity of an aminoglycoside antibiotic.

European Patent Office

# EUROPEAN SEARCH REPORT

Application Number

EP 91114479.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB - A - 2 139 087 (BRISTOL-MYERS COMPANY) * Totality * -- | 1-8 | A 61 K 37/02<br>A 61 K 31/70 |
| P,A | EP - A - 0 388 961 (BRISTOL-MYERS SQUIBB COMPANY) * Claims 1,2,7 * -- | 1,6 | |
| A | US - A - 4 892 733 (BICHON ET AL.) * Abstract; claims 1,7,10,11, 16-20 * ---- | 1 | |

TECHNICAL FIELDS SEARCHED (Int. Cl.5)

A 61 K

The present search report has been drawn up for all claims

| Place of search | Date of completion of the search | Examiner |
|---|---|---|
| VIENNA | 26-11-1991 | AUGUSTIN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document